# FASCICULE DE BREVET EUROPEEN

(11) **EP 1 261 629 B1**
(45) Date de publication et mention de la délivrance du brevet: **02.11.2006**
(21) Numéro de dépôt: 01913946.8
(22) Date de dépôt: 07.03.2001
(51) Int. Cl.: C07K 14/04, C12N 15/46, C12N 5/10, C12N 15/67, A61K 39/15

(54) **PARTICULES PSEUDOVIRALES DE ROTAVIRUS ET LEUR UTILISATION POUR VECTORISER DES PROTEINES OU DES ACIDES NUCLEIQUES**
PSEUDOVIRUS-PARTIKEL DES ROTAVIRUS UND IHRE VERWENDUNG ALS VEKTOREN FÜR PROTEINE UND NUKLEINSÄUREN
ROTAVIRUS PSEUDOVIRAL PARTICLES AND USE THEREOF FOR VECTORIZING PROTEINS OR NUCLEIC ACIDS

(30) Priorité: 07.03.2000 FR 0002892
(43) Date de publication de la demande: 04.12.2002
(73) Titulaire: INSTITUT NATIONAL DE LA RECHERCHE AGRONOMIQUE (INRA), 75007 Paris Cédex 07 (FR)
(72) Inventeur: COHEN, Jean, F-75014 Paris (FR); PONCET, Didier, F-91440 Bures-sur-yvette (FR); CHARPILIENNE, Annie, F-78180 Montigny-le-Bretonneux (FR)
(74) Mandataire: Vialle-Presles, Marie José
(86) Numéro de dépôt international: PCT/FR2001/000676
(87) Numéro de publication internationale: WO 2001/066566

(56) Documents cités:
- WO-A-00/26380
- US-A- 5 374 426
- US-A- 5 667 782
- S.E. CRAWFORD ET AL.: "Characterization of Virus-Like Particles Produced by the Expression of Rotavirus Capsid Proteins in Insect Cells" JOURNAL OF VIROLOGY., vol. 68, no. 9, septembre 1994 (1994-09), pages 5945-5952, XP002154676 ICAN SOCIETY FOR MICROBIOLOGY US cité dans la demande

## Description

L'invention est relative à des particules pseudovirales dérivées du rotavirus, et à leurs utilisations.

Les rotavirus sont responsables de près de la moitié des diarrhées néonatales de l'enfant et des jeunes animaux. Chez l'homme, ils sont responsables d'une mortalité importante dans les pays en voie de développement (près de 900 000 enfants/an) et d'une morbidité élevée dans les pays développés. Dans le cas des animaux d'élevage, l'impact économique des rotavirus du veau et du porcelet est considérable.

Les rotavirus sont des virus non-enveloppés, à capside icosaédrique (T=13, gauche). Cette capside est constituée de 3 couches protéiques [ESTES et COHEN, Microbiology Review, 53, 410-449, (1989) ; MATTION *et al.,* Viral infections of the gastrointestinal tract. In A. Kapikian (ed.), Marcel Dekker Inc., New York (1994)].

La couche externe est constituée des protéines VP7 (34 kd) et VP4 (88 kd). VP4 est le constituant des spicules situées à la périphérie du virion ; elle est clivée par la trypsine en 2 sous-unités, dénommées VP5* et VP8* ; elle intervient dans la fixation du virus aux récepteurs cellulaires et dans l'activité hémaglutinine. Par élimination de cette couche externe, on obtient des particules virales à 2 couches (Double Layered Particles : DLP) non-infectieuses en culture cellulaire.

La couche intermédiaire est constituée de la protéine VP6. Cette protéine de 44 kDa représente 50% de la masse du virion. Elle est fortement immunogène et porte des déterminants antigéniques de groupe et de sous-groupe. D'autre part, son élimination entraîne la perte de l'activité transcriptase des particules virales.

Le coeur de la particule virale, qui résulte de l'élimination de VP6 à partir des DLP comprend la protéine VP2 (90 kd), qui entoure l'ARN génomique et 2 protéines minoritaires : VP1 (125 kd) et VP3 (90 kd), possédant respectivement une activité ARN polymérase et une activité guanylyltransférase [ESTES et COHEN, Microbiology Review, 53, 410-449, (1989)]. Outre son rôle structural, VP2 est capable de fixer les acides nucléiques, et participe à l'empaquetage de l'ARN viral.

Des travaux antérieurs de l'équipe des Inventeurs ont montré que la protéine VP2, exprimée en absence de toutes les autres protéines virales, s'auto-assemble en particules morphologiquement identiques au coeur [LABBE et al., Journal of Virology, 65, 2946-2952, (1991)]. Les protéines VP6 et VP2 peuvent s'assembler pour donner des particules pseudovirales (Virus Like Particles : VLP) dépourvues d'acide nucléique et donc non-infectieuses, [LABBE et al., Journal of Virology, 65, 2946-2952, (1991)].

Les quatre protéines de la capside (VP2, VP6, VP7 et VP4) peuvent aussi s'assembler pour donner des particules pseudovirales. Les VLP contenant les quatre protéines de capside (VLP2/6/7/4) ont des propriétés semblables à celles des virus infectieux en ce qui concerne l'attachement sur des cellules sensibles [CRAWFORD *et al*., Journal of Virology, 68, 5945-5922, (1994)], et la pénétration intracellulaire [LIPRANDI et al., Virology, 237, 430-438, (1997)].

Il a été proposé d'utiliser des pseudocapsides virales comme vecteurs de molécules d'intérêt biologique, notamment des peptides ou des acides nucléiques.

Par exemple, en vue de la préparation de vaccins, des protéines chimériques résultant de l'insertion de séquences de peptides antigéniques hétérologues dans la protéine HBcAg du virus HBV ont été obtenues. Ces protéines chimériques peuvent s'assembler en particules pseudovirales, à condition que la taille des séquences insérées ne soit pas trop importante. Dans le cas de séquences hétérologues de plus grande taille, les particules pseudovirales peuvent aussi être obtenues par assemblage d'unités constituées par les protéines chimériques avec des unités constituées par la protéine HBcAg [KOLETZKI et al., Journal of Général Virology, 78, 2049-2053, (1997)].

Des particules pseudovirales dérivées de papillomavirus ont également été utilisées pour l'encapsidation d'ADN hétérologue, et son introduction dans une cellule-hôte [TOUZE et COURSAGET, Nucleic Acids Research., 26, 1317-1323, (1998)].

En ce qui concerne les rotavirus, REDMOND *et al*., [Molecular Immunology, 28, 269-278, (1991)] ou FRENCHICK et al., [Vaccine, 10, 783-791, (1992)] décrivent l'utilisation de particules pseudovirales produites par assemblage d'unités VP6 de rotavirus, comme vecteurs de peptides antigéniques de petite taille, faiblement immunogènes. Le peptide antigénique dérivé de la protéine VP4 est fixé -non covalement à VP6, de manière à être présenté à la surface externe de la particule. Les particules pseudovirales ainsi formées jouent un rôle d'immunoadjuvant, augmentant la réponse immunitaire vis-à-vis du peptide antigénique. Cependant, la présentation du peptide antigénique à la surface externe de la particule, favorable à la réponse immunitaire contre ce peptide, présente en revanche l'inconvénient d'exposer celui-ci à la dégradation, notamment dans le cas d'administration *in vivo.*

Les Inventeurs sont maintenant parvenus à obtenir des particules pseudovirales dérivées de rotavirus, permettant l'encapsidation de protéines ou d'acide nucléique, leur administration *in vivo*, et leur vectorisation notamment vers les tissus ou cellules cibles des rotavirus, telles que les entérocytes.

Ils ont en effet constaté que la protéine VP2 entière ou délétée de son extrémité N-terminale, pouvait être fusionnée à une protéine hétérologue, et que des protéines chimériques ainsi obtenues pouvaient s'assembler entre elles, et/ou avec des protéines VP2 natives et/ou des protéines VP6, ainsi qu'avec les protéines externes VP7 et VP4 pour reconstituer des particules pseudovirales fonctionnelles, et notamment possédant des propriétés similaires à celles du virus pour ce qui concerne le ciblage et les interactions précoces avec la cellule.

La présente invention a pour objet une protéine de fusion comprenant une région A constituée par la protéine VP2 d'un rotavirus, ou par un fragment de ladite protéine comprenant au moins une séquence homologue de celle du fragment 121-880 de la protéine VP2 de la souche bovine RF de rotavirus, liée à une région B comprenant un polypeptide d'intérêt I, ladite région B étant fusionnée à l'extrémité N-terminale de ladite région A.

On définit ici comme : « séquence homologue de celle d'un fragment d'une protéine VP de la souche bovine RF de rotavirus » la portion de séquence d'une protéine VP de rotavirus présentant le meilleur alignement avec la séquence complète dudit fragment. La séquence complète de la protéine VP2 de la souche bovine RF a été publiée par [KUMAR *et al.,* Nucleic Acids Res., 17, 2126, (1989)]. Des séquences homologues de n'importe quel fragment de cette séquence peuvent facilement être identifiées par l'homme du métier à l'aide de logiciels de comparaison de séquences, tels que BLAST [ALTSCHUL *et al.,* Nucleic Acids Res., 25, 3389, (1997)]. Des fragments homologues du fragment 121-880 de la protéine VP2 de la souche bovine RF sont ainsi, par exemple : le fragment 121-881 de la protéine VP2 de la souche bovine UK ; le fragment 122-882 de la protéine VP2 de la souche SA11 de rotavirus simien ; le fragment 129-890 de la protéine VP2 de la souche WA de rotavirus humain ; le fragment 138-897 de la protéine VP2 de la souche PO-13 de rotavirus aviaire ; le fragment 124-871 de la protéine VP2 de la souche Cowden de rotavirus porcin.

Selon un mode de réalisation préféré de la présente invention, la région A est constituée par un fragment de la protéine VP2 d'un rotavirus, comprenant au moins une séquence homologue de celle du fragment 93-880 de la protéine VP2 de la souche bovine RF.

La taille du polypeptide d'intérêt I peut varier de quelques acides aminés à quelques centaines d'acides aminés. Il peut s'agir par exemple d'un antigène, notamment d'un antigène viral ou bactérien contre lequel on souhaite induire une réponse au niveau de la muqueuse intestinale ; d'une enzyme, destinée à supplémenter une fonction déficiente au niveau des entérocytes, etc. Il peut également s'agir d'un polypeptide comprenant un domaine peptidique de liaison à un acide nucléique, capable de reconnaître spécifiquement une séquence-cible d'ADN ou d'ARN, permettant ainsi la fixation à une protéine chimérique conforme à l'invention d'une séquence d'acide nucléique comprenant ladite séquence-cible, et son encapsidation dans une particule pseudovirale comprenant ladite protéine chimérique.

A titre d'exemples de polypeptides comprenant un domaine peptidique de liaison à un acide nucléique, et pouvant faire partie d'une protéine chimérique conforme à l'invention on citera notamment :
- des protéines d'origine virale ou des fragments de celles-ci comprenant des séquences d'encapsidation. A titre d'exemples non-limitatifs de protéines d'origine virale comprenant un domaine de liaison à l'ARN, on peut citer la protéine de capside du phage MS2, la protéine N du virus de la rage, la protéine NCp7 des lentivirus, la protéine NSP3 des rotavirus. A titre d'exemples non-limitatifs de protéines d'origine virale comprenant un domaine de liaison à l'ADN, on peut citer les protéines intervenant dans l'encapsidation du génome viral, telles que la protéine ICP 8 du virus *Herpes simplex,* la protéine gpNu1 du phage lambda, ou la protéine de liaison à l'ADN des adénovirus.
- des facteurs de régulation en *trans* de la transcription, ou des fragments de ceux-ci comprenant un domaine de liaison à l'ADN. On citera par exemple le trans-activateur du promoteur du gène lacI ou des doigts à zinc naturels ou artificiels [WU et al., Proceedings National Academy Science USA, 92, 344-8, (1995)].

La fixation d'une séquence d'acide nucléique à une protéine chimérique conforme à l'invention comprenant un domaine peptidique de liaison à un acide nucléique peut s'effectuer :
- dans le cas d'une séquence d'ARN, par co-expression dans une même cellule-hôte, d'une séquence d'ADN codant ladite protéine chimérique, et d'une séquence d'ADN pouvant être transcrite en un ARN comprenant une séquence cible reconnue par le domaine peptidique de liaison à un acide nucléique de ladite protéine chimérique ; ou
- dans le cas d'une séquence d'ADN, par transfection avec ladite séquence, des cellules où sont assemblées les pseudo particules, ou par assemblage *in vitro* des protéines constituants les protéines des pseudo particules.

La présente invention a également pour objet les particules pseudovirales de rotavirus comprenant une ou plusieurs protéine(s) chimérique(s) conforme(s) à l'invention.

Les particules pseudovirales conformes à l'invention peuvent comprendre des sous-unité(s) VP2 constituées uniquement de protéines chimériques conformes à l'invention, identiques, ou différant entre elles par la nature de la région B, et notamment du polypeptide d'intérêt I ; elles peuvent également comprendre un mélange de sous-unités VP2 conformes à l'invention, et de sous-unités VP2 natives.

Avantageusement des particules pseudovirales conformes à l'invention comprennent en outre des sous-unités VP6.

Selon un mode de réalisation préféré de la présente invention, une ou plusieurs desdites sous-unités VP6 sont constituées par des protéines chimériques dérivées de la protéine VP6 de rotavirus, par insertion d'une séquence exogène au niveau de la séquence homologue de celle du fragment 200-203 de la protéine VP6 de la souche bovine RF de rotavirus, et/ou au niveau de la séquence homologue de celle du fragment 309-313 de la protéine VP6 de la souche bovine RF de rotavirus.

Ladite séquence hétérologue peut être insérée à l'intérieur de ladite ou desdites région(s), ou en remplacement de tout ou partie de celle(s)-ci.

Les Inventeurs ont en effet constaté, en analysant la structure de VP6 (souche RF), que les 2 régions correspondant aux acides aminés 200-203, et aux acides aminés 309-313 forment 2 boucles, orientées vers le milieu extérieur et qui n'interviennent que faiblement ou pas du tout dans l'assemblage des couches internes et intermédiaires de la capside du rotavirus.

Ces régions permettent l'insertion de séquences peptidiques constituant par exemple : des ligands pour des récepteurs cellulaires, afin de moduler le ciblage des particules pseudovirales, des épitopes qui permettent notamment d'élargir les propriétés antigéniques desdites particules, ou des motifs facilitant leur purification.

Des particules pseudovirales conformes à l'invention peuvent comprendre en outre des sous-unités VP7 et VP4.

La présente invention a également pour objet :
- les séquences d'acide nucléique codant des protéines chimériques conformes à l'invention ;
- les cassettes d'expression, dans lesquelles une séquence d'acide nucléique codant une protéine chimérique conforme à l'invention est associée à des éléments appropriés de contrôle de la transcription, et éventuellement de la traduction ;
- les vecteurs recombinants comprenant au moins une séquence d'acide nucléique conforme à l'invention ;
- les cellules-hôtes transformées par au moins une séquence d'acide nucléique conforme à l'invention, et capables d'exprimer ladite séquence.

Des séquences d'acide nucléique, les cassettes d'expression, et les vecteurs recombinants conformes à l'invention peuvent être obtenus par les techniques classiques du génie génétique, telles que celles décrites par SAMBROOK et al., [MOLECULAR CLONING, A LABORATORY MANUAL, 2nd Ed., Cold Spring Harbor Laboratory Press, Cold Spring Harbor, N.Y., (1989)]. Des éléments de contrôle de la transcription et de la traduction, ainsi que les vecteurs utilisables pour la construction de cassettes d'expression et de vecteurs recombinants conformes à l'invention seront choisis notamment en fonction de la cellule-hôte que l'on souhaite utiliser.

Des cellules-hôtes utilisables pour l'expression de protéines chimériques et la production de particules pseudovirales conformes à l'invention, sont en particulier des cellules eucaryotes, et notamment des cellules d'insectes, par exemple des cellules de *Spodoptera frugiperda.*

Des vecteurs utilisables dans ces cellules d'insectes sont notamment des vecteurs dérivés de baculovirus. Des méthodes de clonage et d'expression de protéines recombinantes dans un système baculovirus/cellules d'insectes, et des vecteurs utilisables pour la mise en oeuvre de ces méthodes sont connus de l'homme de l'art, et sont décrits par exemple dans BACULOVIRUS EXPRESSION VECTORS : A LABORATORY MANUAL Freeman and Cie, New York, (1992). D'autres méthodes et d'autres vecteurs également utilisables sont décrits par exemple dans la Demande EP 0 345 152, dans la Demande EP 0 651 815, ou dans la Demande EP 0 638 647 aux noms de l'INSTITUT NATIONAL DE LA RECHERCHE AGRONOMIQUE et du CENTRE NATIONAL DE LA RECHERCHE SCIENTIFIQUE ainsi que dans la Demande PCT WO 95/20672.

Les mêmes vecteurs sont aussi utilisables pour la production d'ARN dans la cellule-hôte, mais on peut aussi envisager d'utiliser des vecteurs qui comportent le promoteur de l'ARN polymérase du phage T7 (ou de tout autre phage de même type, e.g. T3, SP6). Dans ce dernier cas il conviendra d'utiliser des cellules hôtes où le gène codant cette polymérase virale a été introduit et peut être exprimé conditionnellement ou constitutivement [POLKINGHORNE et ROY, Nucleic Acids Res., 23(1), 188-191, (1995)].

La présente invention a également pour objet un procédé d'obtention de particules pseudovirales conformes à l'invention, caractérisé en ce qu'il comprend la mise en culture d'une cellule-hôte exprimant une séquence d'acide nucléique codant une sous-unité VP2 conforme à l'invention, et la récupération des particules pseudovirales à partir de la culture.

Selon un mode de mise en oeuvre préféré de la présente invention, ladite cellule-hôte exprime en outre au moins une séquence d'acide nucléique choisie(s) parmi :
- une séquence d'acide nucléique codant une sous-unité VP2 native ;
- une séquence d'acide nucléique codant une sous-unité VP6 native ;
- une séquence d'acide nucléique codant une sous-unité VP6 comprenant une séquence hétérologue au niveau de la région correspondant aux acides aminés 200-203, et/ou au niveau de la région correspondant aux acides aminés 309-313 de la VP6 native.

Selon une disposition préférée de ce mode de réalisation, ladite cellule-hôte exprime en outre au moins une séquence d'acide nucléique choisie(s) parmi :
- une séquence d'acide nucléique codant une sous-unité VP7 native ;
- une séquence d'acide nucléique codant une sous-unité VP4 native.

Avantageusement, dans le cas où ladite cellule-hôte exprime une séquence d'acide nucléique codant une sous-unité VP2 conforme à l'invention comprenant un domaine peptidique de liaison à un acide nucléique, elle est en outre transformée par une séquence d'acide nucléique pouvant être transcrite en une molécule d'ARN comprenant la séquence-cible reconnue par ledit domaine peptidique de liaison. Par exemple, ladite cellule-hôte est en outre infectée par un baculovirus recombinant contenant une séquence d'acide nucléique pouvant être transcrite en une molécule d'ARN comprenant une séquence-cible reconnue par ledit domaine peptidique de liaison.

Des particules pseudovirales conformes à l'invention peuvent notamment être utilisées pour la préparation de médicaments, notamment en tant que vecteurs d'antigènes, dans le cadre de l'obtention de vaccins, ou bien pour transporter des molécules d'acides nucléiques utilisables par exemple en thérapie génique.

Elles permettent en effet d'administrer et de véhiculer in vivo des protéines ou des acides nucléiques, en les mettant à l'abri de la dégradation dans les fluides biologiques, et de les cibler sur les cellules souhaitées, notamment les cellules capables de multiplier les rotavirus.

Elles peuvent également être utilisées pour protéger et stabiliser un court segment d'ARN, par exemple une portion du génome d'un virus à ARN. Les particules ainsi construites miment ledit virus à ARN, mais sont absolument non-infectieuses. Elles peuvent être utilisées en tant que témoin positif (traceur) parfaitement calibré dans tout le processus de détection dudit virus, par exemple dans un but de diagnostic, ou de contrôle de l'épuration d'un fluide biologique (par exemple, sérum), ou d'un aliment (par exemple, coquillage). En particulier, lors d'une détection par RT-PCR, il est possible de choisir le fragment d'ARN traceur de façon à ce qu'il soit amplifié à l'aide des mêmes amorces que le virus à détecter, mais qu'il s'en différencie soit par une légère différence de taille, soit par l'adition ou la suppression d'un site pour une endonuléase de restriction.

La présente Invention sera mieux comprise à l'aide du complément de description qui va suivre, qui se réfère à des exemples de préparation et d'utilisation de particules pseudovirales conformes à l'invention. Il doit être bien entendu toutefois que ces exemples sont donnés uniquement à titre d'illustration de l'objet de l'Invention dont ils ne constituent en aucune manière une limitation.

### EXEMPLE 1 : CONSTRUCTION DE PARTICULES PSEUDOVIRALES COMPRENANT UNE PROTEINE HETEROLOGUE FUSIONNEE AVEC UN MUTANT DE DELETION DE LA PROTEINE VP2

Le plasmide pBSRF2 [LABBE *et al.,* Journal of Virology, 65, 2946-2952, (1991)] constitué par la séquence complète du gène de la protéine VP2 du rotavirus bovin (souche RF), insérée dans le plasmide pBluescript (STRATAGENE) est utilisé comme matériel de départ.

Un plasmide codant un mutant de VP2 (VP2Δ92) dépourvu des 92 premiers acides aminés, est construit à partir de pBSRF2, selon le protocole décrit par ZENG et al. [Journal of Virology, 72, 201-208, (1998)]. Ce plasmide dénommé pBS2C24Δ, est utilisé pour la construction de plasmides codant des protéines chimériques conformes à l'invention.

Une construction constituée par la séquence codante de VP2Δ92 précédée du lieur TCTAGAGGATCC a été insérée dans le vecteur pBluescript (STRATAGENE) et dénommée : pBSJA16. Cette même construction a été aussi insérée dans les vecteurs pCDNA3 (INVITROGEN), pVL1392 (INVITROGEN), et pFastBac (LIFE TECHNOLOGY) ce qui conduit aux plasmides dénommés respectivement pCDNA3JA16, pVL1392JA16 et pFastbacJA16.

### i) Construction des vecteurs de transfert

### Fusion avec différents fragments de la protéine F du virus respiratoire syncitial (VRS)

Le plasmide pRSVFA codant pour l'intégralité de la protéine F du VRS (sérotype A souche Long) a été décrit par WERTZ et al. [Journal of Virology, 60, 293-301, (1987)].
* Un fragment d'ADN (construction J100) correspondant à la séquence codant les acides aminés 189290 de la protéine F du virus respiratoire syncitial (VRS), flanquée des sites de restriction SalI et XbaI est obtenu par PCR à partir du plasmide pRSVFA, à l'aide des amplimères :
   deb189 : 5'GAATTCGTCGACATGAGCAAAGTGTTAGACCTCA3' et
   fin290 : 5'CTTAAGTCTAGATGATAGAGTAACTTTGCTGTC3'
   Ce fragment est inséré dans pBSJA16 entre les sites SalI et XbaI. L'ensemble de cette construction est ensuite transféré dans pFastBac entre les sites SalI et KpnI. Le plasmide obtenu est dénommé pFastBac190-289JA16.
* Un fragment d'ADN (construction J261) correspondant à la séquence codant les acides aminés 190-450 de la protéine F du virus respiratoire syncitial (VRS), obtenu par PCR et flanqué des sites de restriction SalI et XbaI a été inséré dans pFastbacJA16 aux sites SalI et XbaI. Le plasmide obtenu est dénommé : pFastbac190-450JA16.
* Un fragment d'ADN (construction J61) correspondant à la séquence codant les acides aminés 215-275 de la protéine F du virus respiratoire syncitial (VRS), flanquée des sites de restriction SphI et BamHI est obtenu par PCR à partir du plasmide pRSVFA, à l'aide des amplimères :
   5'GCATGCGTCGACATGTCAAATATAGAAACTGTG3' et
   5'GGATCCTCTAGAGGACATTAACTTCTTCTG3'
* Un fragment d'ADN (construction J21) correspondant à la séquence codant les acides aminés 420-440 de la protéine F du virus respiratoire syncitial (VRS), flanquée des sites de restriction SphI et BamHI est obtenu par PCR à partir du plasmide pRSVFA, à l'aide des amplimères :
   GCATGCGTCGACATGACTAAATGTACAGCATCC et
   GGATCCTCTAGAATCGCACCCGTTAGAAAA.

Ces 2 derniers fragments ont été introduits dans le plasmide pBSRF2Δ92 puis transférés dans pVL1392 (PHARMINGEN). Ces plasmides obtenus ont été dénommés respectivement pVLJA61 et pVLJA21.

### Fusion avec la Green Fluorescent Protein (GFP)

Un fragment d'ADN correspondant à la séquence codant la GFP (longue de 265 acides aminés), est obtenue à partir du plasmide pEGFPC1 (CLONTECH) par l'action séquentielle des enzymes suivantes : NheI, polymérase de Klenow, XbaI.

Cette construction est introduite entre les sites NotI I (rendu franc par le fragment de Klenow de Pol I) et XbaI du plasmide pVL1392JA16, situés en amont de la séquence codant VP2Δ92. Le plasmide obtenu est dénommé pVLJA16PEGFPC1.

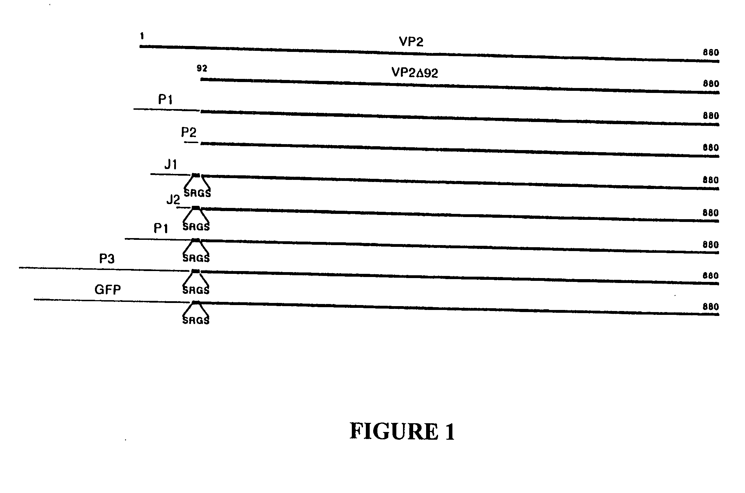

Ces différentes constructions sont schématisées sur la Figure 1.

### ii) Construction des baculovirus recombinants exprimant les protéines chimériques

Chacun des plasmides de transfert décrits ci-dessus est utilisé pour co-transfecter des cellules Sf9 de *Spodoptera frugiperda* avec de l'ADN linéarisé du baculovirus AcNPV. Les baculovirus recombinants sont criblés par la méthode des dilutions limites.

Le baculovirus recombinant comprenant la séquence codant la protéine chimérique J100-VP2Δ92 est dénommé 190-289JA16.

Le baculovirus recombinant comprenant la séquence codant la protéine chimérique J61-VP2Δ92 est dénommé JA61.

Le baculovirus recombinant comprenant la séquence codant la protéine chimérique J21-VP2Δ92 est dénommé JA21.

Le baculovirus recombinant comprenant la séquence codant la protéine chimérique GEP-VP2Δ92 est dénommé GFPJA16.

Le baculovirus recombinant comprenant la séquence codant la protéine chimérique J261-VP2Δ92 est dénommé 190-450JA16.

### Construction de baculovirus recombinants exprimant les protéines VP6, VP7, ou VP4 de rotavirus :

Le baculovirus recombinant dénommé BVP6A exprimant la protéine VP6, est construit comme décrit par TOSSER *et al.* [Journal of Virology., 66(10), 5825-5831, (1992)].

Le baculovirus recombinant dénommé BVP7 A459RD. exprimant la protéine VP7 sous contrôle du promoteur polyèdrine est construit comme décrit par FRANCO *et al.* [Journal of Général Virology, 74, 2579-2586, (1993)].

Le baculovirus recombinant dénommé BVP4 exprimant la protéine VP4 sous contrôle du promoteur polyédrine est construit par clonage dans pBluescript de la séquence codant VP4 obtenue par RT-PCR à partir du génome de la souche RF de rotavirus, et transfert dans le vecteur pVL941.

### EXEMPLE 2 : PRODUCTION ET PURIFICATION DE PARTICULES PSEUDOVIRALES CONFORMES A L'INVENTION

### 1) Particules pseudovirales obtenues par co-expression d'une protéine chimérique conforme à l'invention et de la protéine VP6 de type sauvage :

Des cellules Sf9 sont co-infectées, à une multiplicité d'infection de 5 UFP par cellule pour chaque baculovirus, par l'un des baculovirus recombinants exprimant une protéine VP2 chimérique décrits ci-dessus, et par un baculovirus recombinant exprimant la protéine VP6 (BVP6A). Après 1 h d'incubation à 27°C pour permettre l'adsorption des virus, l'inoculum est retiré et remplacé par du milieu contenant 1% de sérum de veau foetal. Les cellules sont lysées par l'infection et les particules pseudovirales relarguées dans le milieu (5 à 7 jours post-infection) sont purifiées par centrifugation isopycnique sur gradient de chlorure de césium, après clarification du lysat cellulaire et extraction au Fréon 113. On observe une seule bande, correspondant à une densité de 1,30 qui contient les particules pseudo-virales chimériques. La concentration en protéines dans la bande contenant les VLP chimériques est mesuré par la méthode de BRADFORD, avec comme référence la sérum albumine bovine.

L'observation en microscopie électronique d'un échantillon de la préparation purifiée montre la présence de particules pseudo-virales de morphologie identique à celles obtenues par co-expression de la protéine VP2 et de la protéine VP6 de type sauvage. La protéine fusionnée avec VP2Δ92 est située à l'intérieur des particules pseudo-virales.

Dans le cas des particules pseudo-virales contenant la construction GFP-VP2Δ92, l'observation au microscope à fluorescence montre que les particules sont fluorescentes et qu'il est possible de visualiser le signal émanant d'une seule particule pseudo virale.

Le rendement est d'environ 1 à 3 mg de particules pseudo-virales pour 7x10⁸ cellules Sf9.

### 2) Particules pseudovirales obtenues par co-expression d'une protéine chimérique conforme à l'invention et des protéines VP6 et VP7 de type sauvage :

Des cellules Sf9 sont co-infectées à une multiplicité d'infection de 5 UFP par cellule pour chaque baculovirus (GFPJA16, BVP6A, BVP7A459RD), par l'un des baculovirus recombinants exprimant une protéine VP2 chimérique décrits ci-dessus, et par des baculovirus recombinants exprimant les protéines VP6 et VP7 de type sauvage. Les autres étapes de la production et de la purification (y compris le rendement) sont identiques à celles décrites en 1) ci-dessus.

### 3) Particules pseudovirales obtenues par co-expression d'une protéine chimérique conforme à l'invention (GFP-VP2Δ92) et des protéines VP6, VP7 et VP4 de type sauvage:

Des cellules Sf9 sont co-infectées à une multiplicité d'infection de 5 UFP par cellule pour chaque baculovirus (GFPJA16, BVP6A, BVP7A459RD, BVP4), par l'un des baculovirus recombinants exprimant une protéine chimérique VP2 décrits ci-dessus, et par des baculovirus recombinants exprimant les protéines VP6, VP7 et VP4 de type sauvage.

Les cellules sont cultivées et les particules pseudovirales sont récoltées et purifiées selon le protocole suivant : Après 1 h d'incubation à 27°C pour permettre l'adsorption des virus, l'inoculum viral est retiré et remplacé par du milieu à 1% de sérum de veau foetal. Les particules pseudovirales relarguées dans le milieu 7 jours post-infection sont purifiées par centrifugation à travers un coussinet de saccharose à 45%, suivie d'une centrifugation isopycnique sur gradient de chlorure de césium. On observe une seule bande, correspondant à une densité de 1,3. La concentration en particules recombinantes est mesurée comme décrit en 1) ci-dessus. L'observation en microscopie électronique des particules pseudovirales ainsi obtenues montre que leur morphologie et leur stoechiométrie sont identiques à celle des particules pseudovirales obtenues par co-expression des protéines VP2, VP6, VP4, et VP7 de type sauvage. Ces particules chimériques ont les propriétés du virus de type sauvage pour ce qui concerne le ciblage et les interactions précoces avec la cellule.

### EXEMPLE 3 : PROPRIETES IMMUNOLOGIQUES DES ANTIGENES ENCAPSIDES DANS LES PARTICULES PSEUDOVIRALES

Des particules pseudovirales construites à partir des baculovirus 190-450JA16 ou GFPJA16 et du baculovirus VP6A, purifiées comme décrit à l'exemple 2 ci-dessus sont utilisées pour immuniser des souris selon différents protocoles :
- immunisation par voie nasale : les préparations de pseudo-particules, à la concentration d'environ 1 mg/ml, sont administrées à raison de 10 *µ*l par narine de souris. Un rappel dans les mêmes conditions a lieu 21 jours plus tard ;
- immunisation par voie intrapéritonéale. La première immunisation est faite avec 10 *µ*g de pseudo-particules en émulsion avec de l'adjuvant complet de Freund. Le rappel est administré 21 jours plus tard avec la même quantité de pseudo-particules mais avec de l'adjuvant de Freund incomplet.

Les sérums des souris sont prélevés avant immunisation, après la première immunisation, et 7 jours après un rappel, et leur réactivité avec les protéines VP2, VP6 de rotavirus et avec, respectivement, la protéine F du VRS ou la protéine GFP est évaluée comme suit.

### Sérum des souris immunisées avec les particules pseudo virales contenant la construction P1-VP2Δ92

### Réactivité vis-à-vis des protéines de rotavirus :

Les sérums sont testés à des dilutions de 1/100 à 1/500 par immunocoloration après électrophorèse et transfert sur une membrane de PVDF ("western blot") de particules virales purifiées.

### Réactivité vis-à-vis de cellules infectées par le virus respiratoire syncitial (VRS) :

Les sérums sont testés en immunofluorescence à des dilutions de 1/10 à 1/160 sur des cellules Hep 9 infectées par le VRS (souche Long). Dans ces conditions, les sérums hyperimmun sont positifs jusqu'à une dilution supérieure à 1/160 alors que les sérum préimmuns restent négatifs dans toute la gamme des dilutions testées.

### Réactivité vis-à-vis de protéine F recombinante:

La protéine F recombinante utilisée est produite chez le baculovirus [HIMES et GERSHWIN, Journal of General Virology, 73, 1563-1567, (1992)]. Les sérums sont testés en ELISA à des dilutions de 1/50 à 1/1600 selon le protocole suivant : environ 100 ng de protéine F recombinante dans du tampon 0,1 M bicarbonate de sodium sont incubés une nuit dans les puits de micro-plaques, qui sont ensuite saturés pendant 30 minutes avec une solution de blocage (Tris HCl 25 mM, 150 mM NaCl, 0,1% Tween 20, 3% sérum albumine bovine ; en abrégé TBST).

On dépose ensuite dans les puits des dilutions (en TBST) des sérums à tester. Après 1 h d'incubation et rinçage on révèle la présence des anticorps spécifiques grâce à des anticorps anti-H+L de souris conjugués à la phosphatase alcaline. Dans ces conditions, le titre des sérums après le rappel est supérieur à 1/400, alors que les sérums préimmuns sont négatifs pour toutes les dilutions testées.

### Sérums des souris immunisées avec les particules pseudovirales contenant la construction GFP-VP2Δ92

### Réactivité vis-à-vis des protéines de rotavirus :

Des particules purifiées de rotavirus sont analysées par électrophorèse en gel de polyacrylamide, et les protéines une fois séparées sont transférées sur membrane de PVDF. Les sérums sont testés par coloration immunochimique pour leur réactivité avec les protéines virales à des dilutions de 1/100 à 1/5000. Jusqu'à une dilution de 1/2000 ils reconnaissent les protéines VP2 et VP6. Les sérums préimmuns sont négatifs pour toutes les dilutions testées.

### Réactivité vis-à-vis de lysat de E. coli exprimant de la GFP recombinante :

Des bactéries E. coli exprimant de la GFP sont lysées par 1% SDS et 50 mM DTT. Le lysat obtenu est porté à 100°C et analysé par électrophorèse en gel de polyacrylamide. Les protéines après analyse sont transférées sur une membrane de PVDF. Les sérums sont utilisés à des dilutions de 1/100 à 1/5000 pour une coloration immunochimique ("Western Blot").

Dans ces conditions, les sérums immuns sont positifs jusqu'à une dilution de 1/2000. Les sérums préimmuns sont négatifs pour toutes les dilutions testées.

### EXEMPLE 4: CONSTRUCTION DE PARTICULES PSEUDOVIRALES COMPRENANT UNE PROTEINE VP6 MODIFIEE

La résolution de la structure de VP6 de la souche bovine RF à 2 Angströms, a permis d'identifier 2 boucles qui sont orientées vers le milieu extérieur et qui n'interviennent que faiblement ou pas du tout dans l'assemblage de la capside. Ces 2 boucles correspondent aux acides aminés 200-203 (B1) et 309-313 (B2).

Plusieurs délétions/insertions sont réalisées dans B1 et B2. Elles visent à ajouter les fonctionnalités suivantes aux VLP :
- présentation de l'épitope immunogène M19 de la protéine F du VRS [CHARGELEGUE *et al*., Journal of Virology, 72, 2040-2046, (1998)] à l'extérieur des VLP (Fl) ;
- addition de motifs contenant plusieurs résidus His permettant de faciliter la purification des nanoboîtes (F2) ;
- ciblage vers des récepteurs cellulaires spécifiques (F3).

Le site Bl s'est avéré permissif pour toutes les constructions qui sont réalisées, mais aucune des fonctionnalités F1, F2 ou F3 n'a pu être ajoutée aux VLP. On peut supposer que la région correspondant à Bl n'interviendrait pas du tout dans les interactions trimère-trimère (d'où la grande permissivité du site), mais qu'elle serait dirigée vers l'espace inter-trimères (d'où la faible accessibilité et l'absence de fonctionnalités).

En revanche, le site B2, malgré une permissivité moindre que celle de B1, permet l'insertion de motifs relatifs à la fonctionnalité F3 sans gêner l'assemblage des VLP.

Ainsi, la séquence ATFALRGDNPQ a été ajoutée entre les résidus proline qui occupent les positions 309 et 313 de VP6 par mutation dirigée dans le plasmide pFastbacVP6 à l'aide de 2 oligonucléotides synthétiques et de la trousse QuikChange^{®}, (STRATAGENE). Le plasmide obtenu dénommé pFastbacVP6-21C permet d'obtenir, comme décrit à l'exemple 1 ci-dessus, le baculovirus Bac VP6-21C qui exprime la protéine VP6 mutée. L'infection simultanée de cellules Sf9 par BacVP6-21C et par un baculovirus qui permet l'expression de la protéine VP2 sauvage ou d'une protéine VP2 chimérique conforme à l'invention conduit à l'assemblage de particules pseudovirales. Ces dernières sont purifiées comme indiqué à l'exemple 2 ci-dessus, en gradient isopycnique de CsCl.

La fonctionnalité de la séquence ajoutée dans VP6 a été évaluée par un test de type ELISA : de l'intègrine α5β3 soluble, dont le motif RGD est le ligand, est absorbée dans des puits de micro-plaques. Après saturation des puits avec du tampon TBST, on ajoute soit des particules pseudovirales normales, soit des particules pseudovirales contenant la protéine VP6 chimérique mentionnée plus haut. On détecte les pseudo particules attachées à l'intégrine α5β3 à l'aide d'un sérum de lapin anti-VP6 et d'un conjugué anti-H+L de lapin conjugué à la phosphatase alcaline. Après addition du substrat de l'enzyme, la coloration des puits où ont été déposées des particules pseudovirales contenant la séquence ATFALRGDNPQ est très intense alors que celle où ont été déposées les particules pseudovirales normales reste claire. Ces résultats montrent que les particules pseudovirales chimériques obtenues sont bien reconnues par l'intégrine α5β3. Ceci devrait permettre le criblage des particules pseudovirales vers les populations cellulaires qui possèdent l'intégrine α5β3.

### EXEMPLE 5 : CONSTRUCTION DE PARTICULES PSEUDOVIRALES CONTENANT UN FRAGMENT D'ARN

### Construction d'une VP2 chimérique comprenant le mutant VP2Δ92 fusionné avec le dimère de la protéine de capside du phage MS2

### Fusion du mutant VP2Δ92 avec le dimère de la protéine de capside du phage MS2

Les plasmides pcT21 ou d1-13 contenant une séquence codant pour le dimère de la protéine de capside du phage MS2, ont été décrit par [PEABODY et LIM, Nucleic Acid Research, 24, 2354-2359, (1996)].

Un fragment d'ADN correspondant à la séquence codant le dimère de la protéine de capside du phage MS2, flanquée des sites de restriction NotI et XbaI est obtenu par PCR à partir d'un des plasmides pcT21 ou d1-13. Selon le plasmide employé, on obtient des diméres de la protéine de capside de conformations différentes qui auront donc des affinités différentes pour la séquence cible d'ARN.

Chaque construction est introduite entre les sites NotI et XbaI du plasmide pFastbacJA16 en amont de la séquence codant VP2Δ92. Les plasmides obtenus sont dénommés pFastbacpCT21 et pFastbacd1-13.

### Construction d'un baculovirus recombinant exprimant les protéines chimériques :

Chacun des plasmides pFastbacT21 et pFastbacd1-13 est utilisé comme décrit à l'exemple 1 ci-dessus, pour des baculovirus recombinants. Ces baculovirus recombinants sont respectivement dénommés MS2pCT21JA16 ou MS2d1-13JA16.

Ces baculovirus recombinants peuvent être utilisés pour infecter des cellules d'insectes, comme décrit à l'exemple 1, seuls ou associé à un baculovirus recombinant exprimant la protéine VP6 sauvage ou modifiée, et éventuellement à des baculovirus recombinants exprimant les protéines VP7 et VP4.

Les particules pseudovirales peuvent être purifiées par le protocole décrit dans l'exemple 1 ci-dessus.

### Construction d'un baculovirus recombinant exprimant un ARN pouvant se fixer à la protéine chimérique MS2-VP2Δ92

Cette construction est réalisée dans pFastbac en insérant entre les sites BamHI et EcoRI un oligonucléotide synthétique correspondant à la séquence cible de MS2 (par exemple : 5'ACAUGAGGAUUACCCAUGG3' ou des répétitions de cette séquence).

Dans un deuxième temps on introduit entre les sites SalI et PstI la séquence :
CGAGTA GGAACGAGGG TACAGCTTCC TTCTTTTCTG TCTCTGTTTA
GATTATTTTA ATCACCATTT AAAATTGATT TAATCAGAAG C
utilisable pour le diagnostic d'un astrovirus. Le plasmide ainsi obtenu pFastbacMs2-As est utilisé pour obtenir le baculovirus BacMs2-As.

Ce baculovirus recombinant BacMs2-As, le baculovirus recombinant MS2T21JA16 (ou MS2d1-13JA16) et un baculovirus recombinant exprimant 1a protéine VP6 sauvage ou modifiée sont utilisés pour co-infecter des cellules d'insectes, comme décrit à l'exemple 1. Il est possible d'ajouter lors de la co-infection les baculovirus recombinants exprimant les protéines VP7 et VP4. Les particules pseudovirales peuvent être purifiées par le protocole décrit dans l'exemple 1 ci dessus. Les particules ayant incorporé l'ARN comprenant la séquence-cible peuvent être distinguées de celles n'ayant pas incorporé l'ARN sur la base de leur densité en gradient de CsCl qui est plus élevée dans le premier cas.

### EXEMPLE 6 : CONSTRUCTION DE PARTICULES PSEUDOVIRALES COMPRENANT UNE PROTEINE HOMOLOGUE FUSIONNEE AVEC UN MUTANT DE DELETION DE VP2

Un plasmide comprenant une séquence codant pour la protéine VP4 d'un rotavirus bovin (souche RF) a été modifié de façon à créer un site pour l'enzyme de restriction XbaI à la position de la séquence codante qui correspond au site de clivage VP8/VP5 (résidu Arg₂₄₁). Le fragment d'ADN codant pour la protéine VP8* mature présente à la surface de la particule de rotavirus est récupéré à partir de ce plasmide. Ce fragment d'ADN codant pour VP8* est inséré dans pFastbacJA16, aux sites SalI et XbaI. Le plasmide obtenu est dénommé pFastbacVP8JP16, et utilisé pour construire le baculovirus recombinant BacVP8JA16, qui permet l'expression en cellules d'insecte d'une protéine de fusion constituée de l'intégralité de VP8*, d'un lieur, et de VP2Δ92 (VP8-VP2Δ92).

Les particules pseudovirales obtenues par coexpression de VP8-VP2Δ92 et de VP6 de type sauvage sont produites et purifiées comme indiqué dans l'exemple 2. Le rendement en particules purifiées est identique à celui indiqué dans l'exemple 2.

Par coloration immunochimique après électrophorèse et transfert sur membrane de PVDF (Western blot), à l'aide d'anticorps monoclonaux dirigés contre VP8*, on peut mettre en évidence des épitopes neutralisants conservés dans la protéine chimérique. L'immunisation de souris avec ces particules purifiées induit des anticorps anti-VP8* neutralisant le rotavirus. Les anticorps obtenus sont spécifiques de la souche de rotavirus dont est issu VP8* et leur titre est élevé. Il est également possible d'ajouter la protéine VP7 aux particules pseudovirales ainsi obtenues et d'augmenter l'activité neutralisante des sérums résultant de l'immunisation de souris avec ces particules.

## Revendications

1. Protéine de fusion comprenant une région A constituée par la protéine VP2 d'un rotavirus, ou par un fragment de ladite protéine comprenant au moins une séquence homologue de celle du fragment 121-880 de la protéine VP2 de la souche bovine RF de rotavirus, liée à une région B constituée par un polypeptide hétérologue comprenant un polypeptide d'intérêt I, ladite région B étant fusionnée à l'extrémité N-terminale de ladite région A.

2. Protéine de fusion selon la revendication 1, **caractérisée en ce que** la région B comprend un lieur peptidique L, placé entre la région A et le polypeptide d'intérêt I.

3. Protéine de fusion selon une quelconque des revendications 1 ou 2, **caractérisée en ce que** le polypeptide d'intérêt I est choisi parmi :
- les polypeptides antigéniques ;
- les polypeptides possédant une activité enzymatique ;
- les polypeptides comprenant un domaine peptidique de liaison à un acide nucléique.

4. Particule pseudovirale de rotavirus **caractérisée en ce qu'**elle comprend au moins une protéine de fusion selon une quelconque des revendications 1 à 3.

5. Particule pseudovirale selon la revendication 4, **caractérisée en ce qu'**elle comprend en outre des sous-unités VP6.

6. Particule pseudovirale selon la revendication 5, **caractérisée en ce qu'**une ou plusieurs desdites sous-unités VP6 sont constituées par des protéines chimériques dérivées de la protéine VP6 de rotavirus, par insertion d'une séquence exogène au niveau de la séquence homologue de celle du fragment 200-203 de la protéine VP6 de la souche bovine RF de rotavirus, et/ou au niveau de la séquence homologue de celle du fragment 309-313 de la protéine VP6 de la souche bovine RF de rotavirus.

7. Particule pseudovirale selon une quelconque des revendications 5 ou 6, **caractérisée en ce qu'**elle comprend en outre des sous-unités VP7 et VP4.

8. Séquence d'acide nucléique codant une protéine de fusion selon une quelconque des revendications 1 à 3.

9. Cassette d'expression comprenant une séquence d'acide nucléique selon la revendication 8, associée à des éléments appropriés de contrôle de la transcription, et éventuellement de la traduction.

10. Vecteur recombinant comprenant au moins une séquence d'acide nucléique selon la revendication 8.

11. Vecteur recombinant selon la revendication 10, **caractérisé en ce qu'**il s'agit d'un vecteur dérivé de baculovirus.

12. Cellule-hôte transformée par au moins une séquence d'acide nucléique selon la revendication 8.

13. Cellule-hôte selon la revendication 12, **caractérisé en ce qu'**il s'agit d'une cellule d'insecte.

14. Procédé d'obtention de particules pseudovirales selon une quelconque des revendications 4 à 7, **caractérisé en ce qu'**il comprend la mise en culture d'une cellule-hôte selon une quelconque des revendications 12 ou 13, et la récupération des particules pseudovirales à partir de la culture.

15. Procédé selon la revendication 14, **caractérisé en ce que** ladite cellule-hôte est en outre transformée par une ou plusieurs séquences d'acide nucléique choisie(s) parmi :
- une séquence d'acide nucléique codant une sous-unité VP2 native ;
- une séquence d'acide nucléique codant une sous-unité VP6 native ;
- une séquence d'acide nucléique codant une sous-unité VP6 comprenant une séquence exogène au niveau de la séquence homologue de celle du fragment 200-203 de la protéine VP6 de la souche bovine RF de rotavirus, et/ou au niveau de la séquence homologue de celle du fragment 309-313 de la protéine VP6 de la souche bovine RF de rotavirus.

16. Procédé selon la revendication 15, **caractérisé en ce que** ladite cellule-hôte est en outre transformée par une ou plusieurs séquences d'acide nucléique choisie(s) parmi :
- une séquence d'acide nucléique codant une sous-unité VP7 native ;
- une séquence d'acide nucléique codant une sous-unité VP4 native.

17. Procédé selon une quelconque des revendications 14 à 16, **caractérisé en ce que** ladite cellule-hôte, transformée par au moins une séquence d'acide nucléique codant une protéine de fusion selon la revendication 3 comprenant un domaine peptidique de liaison à un acide nucléique, est en outre transformée par une séquence d'acide nucléique pouvant être transcrite en une molécule d'ARN comprenant une séquence-cible reconnue par ledit domaine peptidique de liaison.

18. Utilisation de particules pseudovirales selon une quelconque des revendications 4 à 7, pour l'obtention d'un médicament.

19. Utilisation selon la revendication 18, **caractérisée en ce que** ledit médicament est un vaccin.

20. Utilisation selon la revendication 18, **caractérisé en ce que** ledit médicament est un vecteur de thérapie génique.

## Claims

1. Fusion protein comprising a region A consisting of the VP2 protein of a rotavirus, or of a fragment of said protein comprising at least one sequence which is homologous to that of fragment 121-880 of the VP2 protein of the bovine rotavirus strain RF, linked to a region B consisting of a heterologous polypeptide comprising a polypeptide of interest I, said region B being fused to the N-terminal end of said region A.

2. Fusion protein according to claim 1, **characterised in that** the region B comprises a peptide linker L placed between region A and the polypeptide of interest I.

3. Fusion protein according to any one of claims 1 or 2, **characterised in that** the polypeptide of interest I is selected from among:
- antigenic polypeptides;
- polypeptides having an enzymatic activity;
- polypeptides comprising a peptide linker domain for linking to a nucleic acid.

4. Pseudoviral rotavirus particle, **characterised in that** it comprises at least one fusion protein according to any one of claims 1 to 3.

5. Pseudoviral particle according to claim 4, **characterised in that** it further comprises VP6 subunits.

6. Pseudoviral particle according to claim 5, **characterised in that** one or more of said VP6 subunits consist(s) of chimeric proteins derived from rotavirus VP6 protein, by insertion of an exogenous sequence at the position of the sequence which is homologous to that of fragment 200-203 of the VP6 protein of the bovine rotavirus strain RF, and/or at the position of the sequence which is homologous to that of fragment 309-313 of the VP6 protein of the bovine rotavirus strain RF.

7. Pseudoviral particle according to any one of claims 5 or 6, **characterised in that** it further comprises VP7 and VP4 subunits.

8. Nucleic acid sequence coding for a fusion protein according to any one of claims 1 to 3.

9. Expression cassette comprising a nucleic acid sequence according to claim 8, associated with appropriate transcription and possibly translation control elements.

10. Recombinant vector comprising at least one nucleic acid sequence according to claim 8.

11. Recombinant vector according to claim 10, **characterised in that** it is a vector derived from baculovirus.

12. Host cell transformed by at least one nucleic acid sequence according to claim 8.

13. Host cell according to claim 12, **characterised in that** it is an insect cell.

14. Method of obtaining pseudoviral particles according to any one of claims 4 to 7, **characterised in that** it comprises culturing a host cell according to either of claims 12 or 13 and recovering pseudoviral particles from the culture.

15. Method according to claim 14, **characterised in that** said host cell is furthermore transformed by one or more nucleic acid sequences selected from among:
- a nucleic acid sequence coding for a native VP2 subunit;
- a nucleic acid sequence coding for a native VP6 subunit;
- a nucleic acid sequence coding for a VP6 subunit comprising an exogenous sequence at the position of the sequence which is homologous to that of fragment 200-203 of the VP6 protein of the bovine rotavirus strain RF, and/or at the position of the sequence which is homologous to that of fragment 309-313 of the VP6 protein of the bovine rotavirus strain RF.

16. Method according to claim 15, **characterised in that** said host cell is furthermore transformed by one or more nucleic acid sequences selected from among:
- a nucleic acid sequence coding for a native VP7 subunit;
- a nucleic acid sequence coding for a native VP4 subunit.

17. Method according to any one of claims 14 to 16, **characterised in that** said host cell, transformed by at least one nucleic acid sequence coding for a fusion protein according to claim 3 comprising a peptide linker domain for linking to a nucleic acid, is furthermore transformed by a nucleic acid sequence which can be transcribed into an RNA molecule comprising a target sequence recognised by said peptide linker domain.

18. Use of pseudoviral particles according to any one of claims 4 to 7 for obtaining a medicament.

19. Use according to claim 18, **characterised in that** said medicament is a vaccine.

20. Use according to claim 18, **characterised in that** said medicament is a gene therapy vector.

## Patentansprüche

1. Fusionsprotein, umfassend eine Region A, die aus dem VP2-Protein eines Rotavirus oder einem Fragment dieses Proteins besteht, das wenigstens eine Sequenz umfasst, die zu der des Fragments 121-880 des VP2-Proteins des bovinen RF-Rotavirusstamms homolog ist, die mit einer Region B verknüpft ist, die aus einem heterologen Polypeptid besteht, das ein Polypeptid I von Interesse umfasst, wobei die Region B mit dem N-terminalen Ende der Region A fusioniert ist.

2. Fusionsprotein nach Anspruch 1, **dadurch gekennzeichnet, dass** die Region B ein Linkerpeptid L umfasst, das sich zwischen der Region A und dem Polypeptid I von Interesse befindet.

3. Fusionsprotein nach irgendeinem der Ansprüche 1 oder 2, **dadurch gekennzeichnet, dass** das Polypeptid I von Interesse ausgewählt ist aus:
- antigenen Polypeptiden;
- Polypeptiden, die eine enzymatische Aktivität besitzen;
- Polypeptiden, die eine Peptidbindungsdomäne für eine Nukleinsäure umfassen.

4. Pseudovirales Rotaviruspartikel, **dadurch gekennzeichnet, dass** es wenigstens ein Fusionsprotein nach irgendeinem der Ansprüche 1 bis 3 umfasst.

5. Pseudovirales Partikel nach Anspruch 4, **dadurch gekennzeichnet, dass** es ferner VP6-Untereinheiten umfasst.

6. Pseudovirales Partikel nach Anspruch 5, **dadurch gekennzeichnet, dass** ein oder mehrere dieser VP6-Untereinheiten aus chimären Proteinen bestehen, die sich von dem Rotavirus-VP6-Protein durch Insertion einer exogenen Sequenz an der Stelle der Sequenz, die zu der des Fragments 200-203 des VP6-Proteins des bovinen RF-Rotavirusstamms homolog ist, und/oder an der Stelle der Sequenz, die zu der des Fragments 309-313 des VP6-Proteins des bovinen RF-Rotavirusstamms homolog ist, ableiten.

7. Pseudovirales Partikel nach irgendeinem der Ansprüche 5 oder 6, **dadurch gekennzeichnet, dass** es ferner VP7- und VP4-Untereinheiten umfasst.

8. Nukleinsäuresequenz, die für ein Fusionsprotein nach irgendeinem der Ansprüche 1 bis 3 codiert.

9. Expressionskassette, umfassend eine Nukleinsäuresequenz nach Anspruch 8 in Verbindung mit geeigneten Kontrollelementen für Transkription, und gegebenenfalls Translation.

10. Rekombinanter Vektor, umfassend wenigstens eine Nukleinsäuresequenz nach Anspruch 8.

11. Rekombinanter Vektor nach Anspruch 10, **dadurch gekennzeichnet, dass** es sich um einen Baculovirus-Vektor handelt.

12. Wirtszelle, transformiert mit wenigstens einer Nukleinsäuresequenz nach Anspruch 8.

13. Wirtszelle nach Anspruch 12, **dadurch gekennzeichnet, dass** es sich um eine Insektenzelle handelt.

14. Verfahren zur Herstellung von pseudoviralen Partikeln nach irgendeinem der Ansprüche 4 bis 7, **dadurch gekennzeichnet, dass** es die Kultivierung einer Wirtszelle nach irgendeinem der Ansprüche 12 oder 13, und die Gewinnung der pseudoviralen Partikel aus der Kultur umfasst.

15. Verfahren nach Anspruch 14, **dadurch gekennzeichnet, dass** die Wirtszelle ferner mit ein oder mehr Nukleinsäuresequenzen transformiert ist, ausgewählt aus:
- einer Nukleinsäuresequenz, die für eine native VP2-Untereinheit codiert;
- einer Nukleinsäuresequenz, die für eine native VP6-Untereinheit codiert;
- einer Nukleinsäuresequenz, die für eine VP6-Untereinheit codiert, die eine exogene Sequenz an der Stelle der Sequenz, die zu der des Fragments 200-203 des VP6-Proteins des bovinen RF-Rotavirusstamms homolog ist, und/oder an der Stelle der Sequenz, die zu der des Fragments 309-313 des VP6-Proteins des bovinen RF-Rotavirusstamms homolog ist, umfasst.

16. Verfahren nach Anspruch 15, **dadurch gekennzeichnet, dass** die Wirtszelle ferner mit ein oder mehr Nukleinsäuresequenzen transformiert ist, ausgewählt aus:
- einer Nukleinsäuresequenz, die für eine native VP7-Untereinheit codiert;
- einer Nukleinsäuresequenz, die für eine native VP4-Untereinheit codiert.

17. Verfahren nach irgendeinem der Ansprüche 14 bis 16, **dadurch gekennzeichnet, dass** die Wirtszelle, die mit wenigstens einer Nukleinsäuresequenz transformiert ist, die für ein Fusionsprotein nach Anspruch 3 codiert, das eine Peptidbindungsdomäne für eine Nukleinsäure umfasst, ferner mit einer Nukleinsäuresequenz transformiert ist, die in ein RNA-Molekül transkribiert werden kann, das eine Zielsequenz umfasst, die von der Peptidbindungsdomäne erkannt wird.

18. Verwendung von pseudoviralen Partikeln nach irgendeinem der Ansprüche 4 bis 7 zur Herstellung eines Medikaments.

19. Verwendung nach Anspruch 18, **dadurch gekennzeichnet, dass** das Medikament ein Impfstoff ist.

20. Verwendung nach Anspruch 18, **dadurch gekennzeichnet, dass** das Medikament ein Gentherapievektor ist.
